Europäisches Patentamt

European Patent Office

Office européen des brevets

(11) Numéro de publication : **0 497 686 A1**

# (12) DEMANDE DE BREVET EUROPEEN

(21) Numéro de dépôt : 92400219.9

(51) Int. Cl.⁵ : **A61F 2/76**

(22) Date de dépôt : **28.01.92**

(30) Priorité : **28.01.91 FR 9100933**

(43) Date de publication de la demande :
**05.08.92 Bulletin 92/32**

(84) Etats contractants désignés :
**AT BE CH DE DK ES FR GB GR IT LI LU MC NL PT SE**

(71) Demandeur : **PROTEVAL**
**14, rue de Cerçay**
**F-91800 Brunoy (FR)**

(72) Inventeur : **Cros, Claude**
**04 RC, Résidence Rodin, 64, rue de l'Egalité**
**F-92130 Issy Les Moulineaux (FR)**

(74) Mandataire : **Netter, André**
**Cabinet NETTER, 40, rue Vignon**
**F-75009 Paris (FR)**

(54) Dispositif de liaison ajustable pour prothèse de membre.

(57)    Dispositif de liaison destiné à faire partie d'une prothèse d'un membre du corps humain et à relier entre eux de façon ajustable deux composants de cette prothèse, ce dispositif comprenant un corps de base (1) et deux pièces de jonction (2,3) montées sur des vis sans fin (25,30) du corps de base de façon à pivoter, sur une course limitée (a) correspondant à un angle aigu, autour des axes respectifs (9,19) des vis sans fin, sensiblement perpendiculaires entre eux et non concourants, et à se déplacer en translation, selon une course limitée (d), le long des vis sans fin. Des vis de blocage (31,32) s'appuyant sur des surfaces d'appui des pièces de jonction (3) permettent d'immobiliser chaque pièce de jonction par rapport au corps de base dans toute position choisie à l'intérieur de sa course de pivotement et de sa course de translation, et chaque pièce de jonction est munie de moyens de liaison (42,44,47) à un composant respectif de la prothèse, placés du côté de son propre axe de pivotement opposé à l'autre axe de pivotement.

FIG.1

EP 0 497 686 A1

Jouve, 18, rue Saint-Denis, 75001 PARIS

L'invention concerne les prothèses des membres du corps humain.

Une telle prothèse est habituellement formée de plusieurs composants jouant chacun un rôle déterminé dans le fonctionnement de la prothèse, fabriqués séparément et assemblés entre eux, certains de ces composants fonctionnels pouvant être fabriqués en série et d'autres devant être réalisés sur mesure, par exemple l'emboîture destinée à coiffer le moignon du membre à remplacer. Dans certains cas, un ajustement de la position relative de deux composants successifs, par exemple une emboîture crurale et un genou prothétique de série, par des réglages dits d'angulation et de translation est nécessaire pour obtenir la morphologie correcte de la prothèse. Habituellement, pour tenir compte de cet ajustement, ou adaptation, on stabilise la position relative des deux composants, déterminée expérimentalement, par un dispositif provisoire appelé "coupling" qui vient en prise extérieurement sur ceux-ci, on sépare la prothèse du sujet, on fixe séparément les deux composants sur un même support de travail, on retire le dispositif de liaison provisoire et on le remplace par une pièce de jonction immobilisant de façon définitive les deux composants l'un par rapport à l'autre, obtenue par exemple par moulage in situ.

Cette manière de procéder comporte divers inconvénients. Elle est complexe. La pièce de jonction doit être réalisée à la demande au moment de l'assemblage de la prothèse, et ne peut être fabriquée à l'avance ni en série. Il n'est plus possible, lorsque la prothèse est définitivement assemblée, de modifier les réglages d'angulation et de translation pour tenir compte d'une évolution morphologique chez l'utilisateur, ou pour corriger une erreur lors de l'adaptation initiale.

Le but de l'invention est de remédier à ces inconvénients.

L'invention a pour objet un dispositif de liaison destiné à faire partie d'une prothèse d'un membre du corps humain et à relier entre eux de façon ajustable deux composants de cette prothèse, ce dispositif comprenant un corps de base et deux pièces de jonction montées sur le corps de base de façon à pivoter, sur une course limitée correspondant à un angle aigu, autour d'axes respectifs sensiblement perpendiculaires entre eux et non concourants, et à se déplacer en translation, selon une course limitée, le long des mêmes axes respectifs, des moyens étant prévus pour immobiliser chaque pièce de jonction par rapport au corps de base dans toute position choisie à l'intérieur de sa course de pivotement et de sa course de translation, et chaque pièce de jonction étant munie de moyens de liaison à un composant respectif de la prothèse, placés du côté de son propre axe de pivotement opposé à l'autre axe de pivotement.

Dans la prothèse définitivement assemblée, les deux composants sont solidaires respectivement des deux pièces de jonction. Les mouvements de pivotement et de translation de celles-ci par rapport au corps de base assure donc les réglages d'angulation et de translation entre les composants, ce qui dispense d'effectuer ces réglages préalablement à l'assemblage définitif de la prothèse. De plus, le corps de base et les pièces de jonction peuvent être fabriquées à l'avance et en série. Les inconvénients ci-dessus sont donc entièrement supprimés.

Des caractéristiques optionnelles avantageuses de l'invention sont énoncées ci-après :

– La course de pivotement des pièces de jonction est d'environ 24° ;

– La course de translation des pièces de jonction est d'environ 15 mm ;

– Le corps de base et les pièces de jonction comportent des moyens de guidage respectifs de leurs mouvements mutuels, ces moyens de guidage étant communs au mouvement de pivotement et au mouvement de translation ;

– Les moyens de guidage comprennent deux tiges filetées ayant pour axes respectivement les axes de pivotement des deux pièces de jonction, montées sur le corps de base de façon rotative et immobile en translation, et des trous filetés traversants conjugués des tiges filetées, ménagés dans les pièces de jonction ;

– Les moyens pour immobiliser une pièce de jonction comprennent deux vis s'engageant dans des trous filetés du corps de base, dont les axes sont perpendiculaires à l'axe de pivotement de la pièce de jonction, ces vis s'appuyant sur des surfaces d'appui respectives de celle-ci tournées sensiblement à l'opposé l'une de l'autre ;

– Chaque tige filetée de guidage est montée dans deux paliers solidaires du corps de base, de part et d'autre du trou fileté de la pièce de jonction, l'un de ces paliers étant défini par une douille rapportée dans un trou traversant du corps de base dont le diamètre est supérieur au diamètre extérieur du filetage de la tige de façon à permettre le passage de celle-ci lors du montage du dispositif ;

– Les surfaces d'appui sont planes et parallèles à l'axe de pivotement ;

– Chaque surface d'appui est sensiblement perpendiculaire à l'axe de la vis correspondante, dans la position médiane de la course angulaire de la pièce de jonction ;

– L'une au moins des pièces de jonction présente une face plane de réception pour le composant de prothèse correspondant, tournée à l'opposé de son axe de pivotement et parallèle à celui-ci ;

– L'une au moins des pièces de jonction présente un logement cylindrique de réception pour une partie cylindrique du composant de prothèse correspondant, ce logement ayant un axe perpendiculaire à l'axe de pivotement de la pièce de jonction et s'ouvrant à l'opposé de celui-ci ;

– L'une au moins des pièces de jonction présente un trou fileté d'axe perpendiculaire à son axe de pivotement, s'ouvrant à l'opposé de celui-ci ;
– Le corps de base présente une symétrie par rapport à un axe, qui fait se correspondre mutuellement les deux axes de pivotement.

L'invention vise également un jeu de pièces pour la réalisation d'un dispositif tel que défini ci-dessus, formé d'un corps de base et d'au moins trois pièces de jonction, munis de moyens de guidage mutuel en pivotement et en translation et de moyens d'immobilisation, chacune des pièces de jonction étant munie de moyen de liaison, de formes différentes pour au moins deux d'entre elles, pour des composants de prothèses devant être reliés entre eux, deux quelconques de ces pièces de jonction au choix pouvant être assemblées au corps de base selon la nature des composants à relier.

Ainsi, un corps de base commun et le cas échéant des pièces de jonction communes peuvent être utilisés pour différents types de prothèses, ce qui conduit à une rationalisation de la fabrication.

D'autres caractéristiques et avantages de l'invention ressortiront de la description détaillée ci-après, et des dessins annexés dans lesquels :
– la figure 1 est une vue en coupe d'un dispositif selon l'invention, selon la ligne I-I de la figure 2 ;
– la figure 2 est une vue en coupe selon la ligne II-II de la figure 1 ; et
– la figure 3 est une vue en coupe, correspondant à la figure 2, d'une pièce de jonction alternative utilisable dans le dispositif.

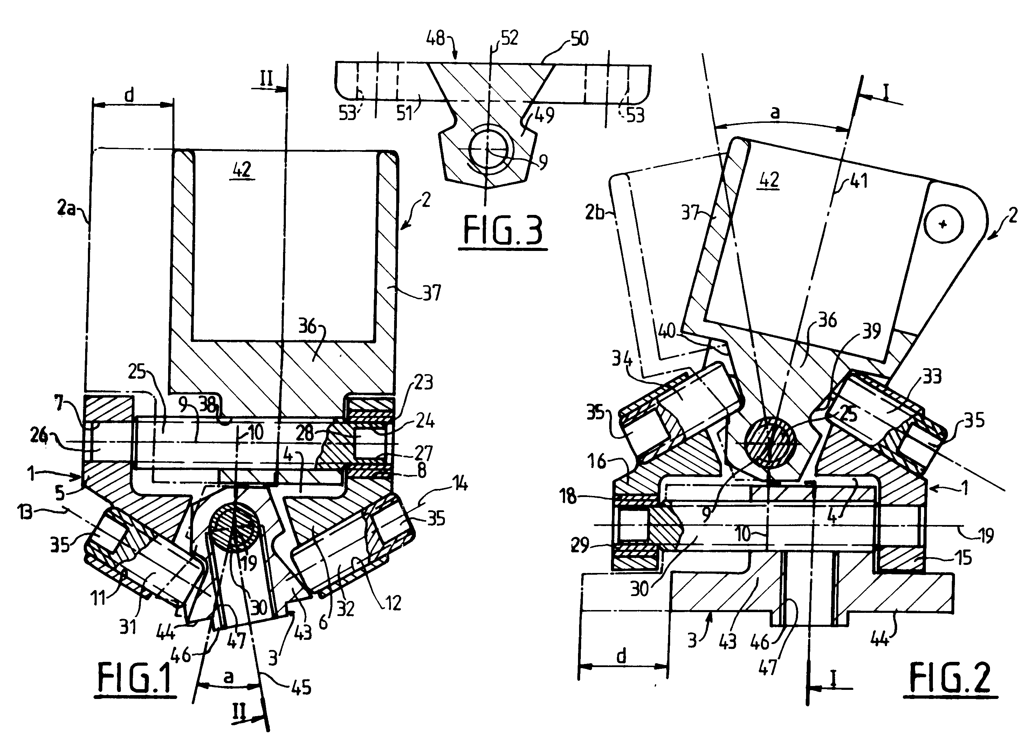

Le dispositif illustré aux figures 1 et 2 comprend essentiellement un corps de base 1 et deux pièces de jonction 2 et 3. Le corps 1 a une forme annulaire irrégulière dont la cavité centrale 4 débouche vers le haut et vers le bas des figures. Il comporte quatre zones fonctionnelles disposées autour de la cavité 4 à des distances angulaires de 90° les unes des autres, et symétriquement deux à deux par rapport à un axe 10. Dans deux de ces zones 5 et 6, symétriques l'une de l'autre, sont ménagés respectivement un trou lisse 7 et un trou fileté 8 ayant le même axe 9 perpendiculaire à l'axe 10, chacun des trous 7 et 8 débouchant par ses extrémités respectives à l'extérieur du corps et dans la cavité 4. Dans les zones 5 et 6 sont également ménagés deux trous filetés 11 et 12 respectivement, symétriques l'un de l'autre par rapport à l'axe 10. Les trous 11 et 12 sont placés au-dessous des trous 7 et 8 respectivement, comme vu à la figure 1, et leurs axes 13 et 14 sont inclinés de façon à se rapprocher de l'axe 9 vers l'extérieur du corps 11. Le diamètre minimal du trou 8 est supérieur au diamètre du trou 7.

Les deux autres zones fonctionnelles 15 et 16 du corps présentent respectivement deux trous 17 et 18 identiques respectivement aux trous 7 et 8, leur axe 19 passant au-dessous de l'axe 9 de ceux-ci, comme vu aux figures 1 et 2. Dans les zones 15 et 16 sont également ménagés au-dessus des trous 17 et 18 des trous filetés respectivement 21 et 22 identiques aux trous 11 et 12.

Le corps 1 dans son ensemble est symétrique par rapport à un axe perpendiculaire à l'axe 10, passant par le milieu du segment ayant pour extrémité les points d'intersection de l'axe 10 avec les axes 9 et 19, et faisant des angles de 45° avec ces derniers. Cette symétrie fait se correspondre mutuellement les trous 7, 8, 11 et 12 et les trous 17, 18, 21 et 22 respectivement.

Dans le trou 8 est vissée une douille 23 filetée extérieurement, et dont l'alésage 24, dans l'exemple illustré, a le même diamètre que le trou 7. Une vis sans tête 25 comporte une partie filetée dont le diamètre est compris entre celui des alésages 7 et 24 et le diamètre intérieur du filetage du trou 8 et qui se prolonge dans les régions des deux extrémités respectivement par des parties cylindriques lisses 26 et 27 qui sont guidées en rotation dans les alésages 7 et 24. Les extrémités de la partie filetée de la vis 25 viennent sensiblement en butée respectivement sur la paroi de la cavité 4, autour de l'alésage 7, et sur l'extrémité de la douille 23 tournée vers cette même cavité, de façon que la vis puisse tourner librement tout en étant pratiquement immobilisée dans la direction de son axe 9. L'extrémité de la vis 25 placée du côté de la douille 23 présente un logement axial 28 pour une clé hexagonale mâle.

Une douille 29 identique à la douille 23 et une vis 30 identique à la vis 25 sont engagées dans les trous 18 et 17 du corps 1. La vis 30 passe à l'intérieur du triangle isocèle formé par les axes 9, 13 et 14.

Quatre vis sans tête identiques 31, 32, 33, 34, filetées sur toute leur longueur, sont vissées respectivement dans les trous 11, 12, 21 et 22. Ces vis présentent des logements axiaux 35 identiques au logement 28 de la vis 25, débouchant dans leur extrémité tournée vers l'extérieur du corps 1.

La pièce de jonction 2 comprend une portion 36 située sensiblement à l'intérieur de la cavité 4 et servant à son guidage et à son immobilisation par rapport au corps 1, au-dessus de l'axe de symétrie du corps 1, et une portion 37 située à l'extérieur de la cavité 4, au-dessus du corps 1, et destinée à recevoir un composant de prothèse. La portion 36 présente un trou traversant fileté 38 qui coopère avec le filetage de la vis 25. La portion 36 présente également des surfaces d'appui 39 et 40 pour les extrémités tournées vers la cavité 4 des vis 33 et 34 respectivement. Ces surfaces d'appui sont contenues dans des plans respectifs parallèles à l'axe 9 et symétriques l'un de l'autre par rapport à un plan 41 passant par ce dernier, ces deux plans formant entre eux un dièdre aigu ouvert vers le haut. La portion 37 présente un logement cylindrique borgne 42, pour un cylindre, notamment un tube, d'un composant de prothèse, ce logement 42 ayant son axe contenu dans le plan 41 et perpendiculaire à l'axe 9, et débouchant vers le haut. La portion 37 présente également des moyens connus en eux-mêmes pour son serrage sur le cylindre du composant.

La pièce de jonction 3 présente une portion 43 identique à la portion 36 de la pièce 2, dont le trou fileté coopère avec la vis 30, et dont les surfaces d'appui coopèrent avec les vis 31 et 32, et une portion de réception d'un composant de prothèse comprenant une surface plane de réception 44, parallèle à l'axe 19 et perpendiculaire au plan 45 de symétrie mutuelle des surfaces d'appui de la portion 43, et tournée vers le bas, ainsi qu'un bossage 46 en saillie sur la surface 44. De plus, un trou fileté 47, dont l'axe est perpendiculaire au plan de la surface 44 et concourant à l'axe 19, est ménagée à partir du sommet plan du bossage 46 et jusqu'au trou fileté coopérant avec la vis 30.

Le dispositif décrit fonctionne de la façon suivante.

Lorsqu'on fait tourner la vis 25 au moyen d'une clé introduite dans le logement 28, la pièce 2 peut se déplacer en translation le long de la vis entre la position extrême vers la droite représentée en trait plein à la figure 1 et la position extrême vers la gauche représentée partiellement en trait mixte sous la référence 2a, soit une course d. De même la pièce 3 peut être déplacée en translation le long de la vis 30, sur la même course d. Par ailleurs, la vis 25 étant immobile, la pièce 2 peut se déplacer d'un mouvement hélicoïdal le long du filet de celle-ci, entre une position extrême vers la droite de la figure 2, représentée en trait plein, et une position extrême vers la gauche indiquée en trait mixte sous la référence 2b, soit une course angulaire a. L'immobilisation en rotation de la pièce 2 est assurée par le vissage des vis 33 et 34, qui viennent serrer entre elles la portion 36 de la pièce. Celle-ci est en même temps immobilisée en translation, remarque étant faite que, comme il est habituel dans une transmission de mouvement par vis sans fin, le faible pas de la vis 25 empêche celle-ci d'être mise en rotation par un effort axial exercé sur la pièce 2.

De la même façon qui vient d'être décrite, la pièce 3 peut également se déplacer en translation et selon un mouvement hélicoïdal par rapport à la vis 30, sur une course de translation d et une course angulaire a. Ces deux mouvements de translation et ces deux mouvements de pivotement assurent tous les réglages voulus d'angulation et de translation pour la liaison entre les deux composants de prothèse.

La pièce de jonction 2 peut recevoir notamment un segment tibial, et la pièce de jonction 3 un genou prothétique. Cette dernière pièce offre grâce au trou fileté 47 des possibilités de réglage supplémentaires. En effet, lorsque le composant prothétique qui doit être lié à cette pièce de jonction est solidaire d'une tige filetée dont le filetage est conjugué de celui du trou 47, en vissant plus ou moins cette tige filetée dans ce trou on peut faire varier à la fois l'éloignement du composant par rapport au corps 1, et son orientation autour de l'axe du trou 47. La position convenable de la tige filetée peut être stabilisée par un écrou s'appuyant sur le bossage 46. Dans l'exemple illustré, la course a est d'environ 24°, ce qui couvre largement l'ensemble des besoins, et la course d est d'environ 15 mm, ce qui correspond à la grande majorité des besoins tout en permettant de loger facilement le dispositif dans pratiquement n'importe quelle prothèse.

Lors de l'assemblage du dispositif illustré, on introduit la vis 25, par sa partie terminale 26, depuis l'extérieur de la pièce 1 à travers le trou 8 puis dans le trou 38 de la pièce 2, dont la portion 36 est introduite à cet effet dans la cavité 4. On visse alors la vis dans le trou 38, puis on engage la partie 26 dans l'alésage 7. On visse la douille 23 dans le trou 8 en la faisant glisser autour de la partie 27 de la vis 25, jusqu'à ce que cette dernière se trouve pratiquement immobilisée en translation. La douille peut présenter des fentes radiales permettant son vissage au moyen d'un tournevis. Elle peut être collée dans le trou 8 pour rendre l'assemblage définitif. La mise en place de la pièce 3 s'effectue de la même façon.

Dans une variante non représentée, les vis 25 et 30 sont remplacées par des tiges lisses solidaires du corps de base et sur lesquelles les pièces de jonction sont montées de façon pivotante et coulissante. Le réglage fin et le blocage de la position en translation de chaque pièce de jonction sont assurés par deux vis ayant un même axe parallèle à celui de la tige et se vissant l'une vers l'autre dans le corps de base pour s'appuyer respectivement sur les deux faces d'extrémités de la portion 36. le mode d'action de ces vis est semblable à celui des vis 31 à 34. Ce type de guidage est plus robuste que le précédent.

La figure 3 représente une pièce de jonction 48 pouvant remplacer la pièce 2 dans le dispositif des figures 1 et 2. Cette pièce 48 comprend une portion 49 identique à la portion 36 de la pièce 2, ainsi qu'une surface de réception plane 50 en forme de H définie par la face supérieure de la portion 49 et par deux branches 51 se raccordant à celle-ci, qui viennent respectivement de part et d'autre des zones 5 et 6 de la pièce 1 dans la direction de l'axe 9. Chacune des branches 51 est symétrique par rapport au plan de symétrie 52 de la portion 49, le plan de la surface 50 étant perpendiculaire à ce plan de symétrie. Quatre trous traversants 53, dont les axes sont perpendiculaires à la surface 50, sont ménagés au voisinage des deux extrémités des deux branches 51. Ces trous 53 peuvent recevoir des éléments de fixation tels que des vis et constituent avec la surface 50 des moyens de liaison de la pièce 48 à un composant de prothèse tel qu'une emboîture crurale.

Les pièces 2, 3 et 48 ne sont que quelques exemples des pièces de jonction qui peuvent être montées sur le corps de base 1. Il est possible de monter sur ce dernier non seulement deux pièces de jonction différentes, comme décrit, mais également deux pièces identiques par exemple semblables à la pièce 2. Par ailleurs, l'une au moins des pièces de jonction peut présenter des moyens permettant un réglage supplémentaire par déplacement du composant prothétique par rapport à la pièce de jonction. Un tel réglage supplémentaire peut être prévu en particulier pour augmenter l'amplitude d'un réglage déjà assuré par les déplacements mutuels des éléments du dispositif, par exemple le réglage de translation antéro-postérieure. Il est également possible d'augmenter l'amplitude des réglages en combinant deux dispositifs selon l'invention, une pièce de jonction commune à ces deux dispositifs comprenant deux portions de coopération avec les corps de base des deux dispositifs respectivement, ou une pièce de jonction de l'un des dispositifs étant rendue solidaire d'une pièce de jonction de l'autre dispositif.

## Revendications

1. Dispositif de liaison destiné à faire partie d'une prothèse d'un membre du corps humain et à relier entre eux de façon ajustable deux composants de cette prothèse, ce dispositif comprenant un corps de base (1) et deux pièces de jonction (2,3) montées sur le corps de base de façon à pivoter, sur une course limitée (a) correspondant à un angle aigu, autour d'axes respectifs (9,19) sensiblement perpendiculaires entre eux et non concourants, et à se déplacer en translation, selon une course limitée (d), le long des mêmes axes respectifs, des moyens (25,30-34) étant prévus pour immobiliser chaque pièce de jonction par rapport au corps de base dans toute position choisie à l'intérieur de sa course de pivotement et de sa course de translation, et chaque pièce de jonction étant munie de moyens de liaison (42,44,47) à un composant respectif de la prothèse, placés du côté de son propre axe de pivotement opposé à l'autre axe de pivotement.

2. Dispositif selon la revendication 1, caractérisé en ce que la course de pivotement des pièces de jonction est d'environ 24°.

3. Dispositif selon l'une des revendications 1 et 2, caractérisé en ce que la course de translation des pièces de jonction est d'environ 15 mm.

4. Dispositif selon l'une des revendications précédentes, caractérisé en ce que le corps de base et les pièces de jonction comportent des moyens de guidage respectifs (25,38) de leurs mouvements mutuels, ces moyens de guidage étant communs au mouvement de pivotement et au mouvement de translation.

5. Dispositif selon la revendication 4, caractérisé en ce que les moyens de guidage comprennent deux tiges filetées (25,30) ayant pour axes respectivement les axes de pivotement (9,19) des deux pièces de jonction, montées sur le corps de base de façon rotative et immobile en translation, et des trous filetés traversants (38) conjugués des tiges filetées, ménagés dans les pièces de jonction.

6. Dispositif selon la revendication 5, caractérisé en ce que chaque tige filetée de guidage est montée dans deux paliers (7,24) solidaires du corps de base, de part et d'autre du trou fileté (38) de la pièce de jonction, l'un (24) de ces paliers étant défini par une douille (23) rapportée dans un trou traversant (8) du corps de base dont le diamètre est supérieur au diamètre extérieur du filetage de la tige de façon à permettre le passage de celle-ci lors du montage du dispositif.

7. Dispositif selon la revendication 4, caractérisé en ce que les moyens de guidage comprennent deux tiges lisses solidaires du corps de base et sur lesquelles les pièces de jonction sont montées de façon pivotante et coulissante.

8. Dispositif selon l'une des revendications précédentes, caractérisé en ce que les moyens pour immobiliser une pièce de jonction (2) comprennent deux vis (33,34) s'engageant dans des trous filetés (21,22) du corps de base, dont les axes sont perpendiculaires à l'axe (9) de pivotement de la pièce de jonction, ces vis s'appuyant sur des surfaces d'appui respectives (39,40) de celle-ci tournées sensiblement à l'opposé l'une de l'autre.

9. Dispositif selon la revendication 8, caractérisé en ce que les surfaces d'appui sont planes et parallèles à l'axe de pivotement.

10. Dispositif selon la revendication 9, caractérisé en ce que chaque surface d'appui est sensiblement perpendiculaire à l'axe de la vis correspondante, dans la position médiane de la course angulaire de la pièce de jonction.

11. Dispositif selon l'une des revendications précédentes, caractérisé en ce que l'une au moins (3,48) des pièces de jonction présente une face plane (44,50) de réception pour le composant de prothèse correspondant, tournée à l'opposé de son axe de pivotement (19,9) et parallèle à celui-ci.

12. Dispositif selon l'une des revendications précédentes, caractérisé en ce que l'une au moins des pièces de jonction présente un logement cylindrique (42) de réception pour une partie cylindrique du composant de prothèse correspondant, ce logement ayant un axe perpendiculaire à l'axe (9) de pivotement de la pièce de jonction et s'ouvrant à l'opposé de celui-ci.

13. Dispositif selon l'une des revendications précédente, caractérisé en ce que l'une au moins (3) des pièces de jonction présente un trou fileté (47) d'axe perpendiculaire à son axe de pivotement (19), s'ouvrant à l'opposé de celui-ci.

14. Dispositif selon l'une des revendications précédentes, caractérisé en ce que le corps de base présente une symétrie par rapport à un axe, qui fait se correspondre mutuellement les deux axes de pivotement (9,19).

15. Jeu de pièces pour la réalisation d'un dispositif selon l'une des revendications précédentes, formé d'un corps de base (1) et d'au moins trois pièces de jonction (2,3,48), munis de moyens de guidage mutuel (25,30,38) en pivotement et en translation et de moyens d'immobilisation (25,30-34), chacune des pièces de jonction étant munie de moyen de liaison (42,44, 50), de formes différentes pour au moins deux d'entre elles, pour des composants de prothèses devant être reliés entre eux, deux quelconques de ces pièces de jonction au choix pouvant être assemblées au corps de base selon la nature des composants à relier.

FIG.1

FIG.2

FIG.3

Office européen
des brevets

# RAPPORT DE RECHERCHE EUROPEENNE

Numero de la demande

EP 92 40 0219

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y<br>A | DE-U-8 913 280 (KRAEMER)<br>* page 8, ligne 10 - page 11, ligne 33; figures * | 1,2,8,11<br>13-15 | A61F2/76 |
| Y<br>A | US-A-3 273 168 (GARDNER)<br>* colonne 2, ligne 38 - colonne 3, ligne 25; figures 1,2,4,5,7 * | 1,2,8,11<br>15 | |
| A | GB-A-2 141 345 (LIC)<br><br>* abrégé; figures 1-4 * | 1,8-10,<br>12 | |
| A | GB-A-2 162 069 (VESSA) | | |
| A | FR-A-2 530 945 (PROTEOR)<br><br>----- | | |

DOMAINES TECHNIQUES RECHERCHES (Int. Cl.5)

A61F

Le présent rapport a été établi pour toutes les revendications

| Lieu de la recherche | Date d'achèvement de la recherche | Examinateur |
|---|---|---|
| LA HAYE | 25 MARS 1992 | KLEIN C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.............................................................................
& : membre de la même famille, document correspondant

EPO FORM 1503 03.82 (P0402)